(19) Europäisches Patentamt
European Patent Office
Office européen des brevets

(11) Publication number: 0 114 703
B1

(12) **EUROPEAN PATENT SPECIFICATION**

(45) Date of publication of patent specification: **15.10.86**

(21) Application number: **84200017.6**

(22) Date of filing: **09.01.84**

(51) Int. Cl.[4]: **C 07 C 51/12,** C 07 C 53/08, C 07 C 67/36, C 07 C 69/14, B 01 J 23/84, B 01 J 27/18

(54) **Process for the preparation of carboxylic acids and/or esters.**

(30) Priority: **25.01.83 NL 8300258**

(43) Date of publication of application: **01.08.84 Bulletin 84/31**

(45) Publication of the grant of the patent: **15.10.86 Bulletin 86/42**

(84) Designated Contracting States: **BE DE FR GB IT NL**

(56) References cited:
**EP-A-0 031 606**
**EP-A-0 072 055**
**EP-A-0 083 121**
**EP-A-0 097 978**
**GB-A-1 326 014**
**GB-B-1 233 121**
**US-A-4 190 729**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 114 703 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of carboxylic acids and/or esters by carbonylation of alcohols in the presence of a rhodium catalyst, an iodide and/or bromide source and a phosphorus, arsenic or antimony-containing compound as promoter. The invention relates in particular to the preparation of acetic acid and/or methyl acetate by carboxylation of methanol.

From German patent specification No. 1,115,234 it is known that acetic acid or a mixture of acetic acid and methyl acetate can be obtained by reaction of methanol with carbon monoxide in the presence of a cobalt catalyst and an iodine compound. However, a disadvantage of this procedure is that a fairly high reaction temperature (230°C) and a high pressure (642 bar) are required. A process in which by using a rhodium catalyst in the presence of an iodine compound the reaction proceeds at lower temperatures and pressures is disclosed in UK patent specification 1,233,121. The catalyst system preferably contains an alkyl or aryl derivative of trivalent phosphorus, arsenic or antimony as a ligand. Comparison of Example 1 with Example 2 teaches that the reaction rate is almost doubled when instead of a simple rhodium salt ($RhCl_3.3H_2O$) use is made of a rhodium complex $Rh(CO)Cl[(C_6H_5)_3P]_2$ containing triphenylphosphine as ligand. From Example 5 it is seen that the ligand may also be present in excess.

In view of the high cost of rhodium it is important that the catalyst system which eventually is formed in the reaction mixture by interaction of the rhodium compound with carbon monoxide, iodide and other ligands be as active as possible, so that under moderate reaction conditions the carbonylation reaction will proceed at a rate suitable for use in actual practice. It is further of interest that the quantity of iodine compound in the reaction mixture is as small as possible. Not only do iodine compounds have a corrosive action, they are also rather volatile. Such compounds as hydrogen iodide, elementary iodine or methyl iodide leave the reactor during recovery of the reaction product by distillation and therefor they must be recirculated.

Obviously when the process is carried out on a large scale it is of importance that the quantity of material to be recirculated be as small as possible. European patent application 42633 discloses a process in which an iodine-free catalyst system is used; however, in this process the rate at which the carbonylation proceeds is rather low.

It has now surprisingly been found that in the carbonylation of alcohols in the presence of a rhodium catalyst and an iodide and/or bromide source certain compounds of pentavalent phosphorus, arsenic or antimony, which will be defined hereinafter, have a stronger promoter action than the compounds of trivalent phosphorus, arsenic or antimony whhich are used in UK patent specification 1,233,121 and consequently allow a more active catalyst system to form. Moreover, the strong promoter action occurs even in the presence of smaller quantities of iodide and/or bromide than when compounds of trivalent phosphorus, arsenic or antimony are used. Furthermore, under the reaction conditions to be used the compounds of pentavalent phosphorus, arsenic or antimony involved are more stable than the corresponding compounds of trivalent phosphorus, arsenic or antimony. Accordingly, they do not lose their promoter action during use and unlike compounds applied so far, they cause no pollution of the reaction mixture by the formation of by-products.

The invention therefore relates to a process for the preparation of carboxylic acids and/or esters by reaction of an alcohol with carbon monoxide in the presence of a rhodium compound, an iodide and/or bromide source and a phosphorus, arsenic or antimony-containing compound as promoter, characterized in that the reaction is carried out in the presence of a compound of the formula

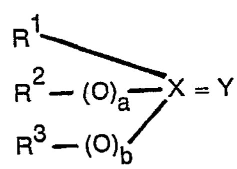

I

wherein X represents phosphorus, arsenic or antimony and Y oxygen, sulphur or selenium, and either a and b, independent of one another, are 0 or 1, $R^1$ represents hydrogen or an unsubstituted or substituted hydrocarbon group and $R^2$ and $R^3$ each represent an unsubstituted or substituted hydrocarbon group, or a and b are 0 and $R^2$ and $R^3$ together with X form a heterocyclic group and $R^1$ represents hydrogen or an unsubstituted or substituted hydrocarbon group, or in the presence of a complex of a compound of formula I with a hydrocarbon iodide or bromide, an acyl iodide or bromide or hydrogen iodide or bromide.

In the process according to the invention first a carboxylic acid is formed which has one carbon atom more than the starting alcohol. The carboxylic acid formed reacts with unconverted alcohol to form an ester, thus producing a mixture of carboxylic acid and ester, the composition of which is dependant, among other things, of the equilibrium of the esterification reaction.

UK patent specification 1,326,014 discloses a process for the carbonylation of alcohols in which a catalyst is used which had been prepared by adding a "stabilizing donor ligand" to a solution of a strong acid, for instance $HBF_4$, and a metal compound having a binuclear metallic cation. In an enumeration of

suitable ligands comprising a very large number of compounds phosphines, phosphine oxides, arsines and stibines are mentioned among others. The only example given relates to the carbonylation of methanol with CO in the presence of a trace of methyl iodide and a catalyst system obtained from $Rh_2(OCOCH_3)_4$, $HBF_4$ and triphenylphosphine. It is stated that the formation of acetic acid was detected. However, no yields are given. In a repeat of this example only a minor quantity of methyl acetate was detected in the reaction mixture. The process according to the invention, wherein the catalyst system is not obtained by adding a ligand to a solution of a strong acid and a rhodium compound, cannot be concluded from this patent specification.

The alcohols used as starting material in the process according to the invention are preferably hydrocarbons substituted with one or more hydroxy groups and optionally with inert substitutes and having 1—20, in particular 1—12 carbon atoms. Examples of suitable alcohols are methanol, ethanol, propanol, isopropanol, butanol, sec.butanol, tert.butanol, the hexanols, the octanols, phenol, benzyl alcohol, ethyleneglycol, glycerol and catechol. Alkanols, particularly those having 1—12, preferably 1—6, carbon atoms are preferred. The alcohol may also be formed in situ from derivatives which under the prevalent reaction condition decompose to form an alcohol. Thus, by hydrolysis with water present in the reaction mixture, alcohols may be formed from ethers.

The choice of the rhodium compound used in the process according to the invention is not very critical. The rhodium compound may be an oxide or an inorganic or organic salt or a complex compound containing one or more ligands such as carbon monoxide, amines, phosphines, arsines, stibines or an olefinically unsaturated compound. Examples of suitable rhodium compounds are $Rh_2O_3$, $RhCl_3$, $RhBr_3$, $RhI_3$, $Rh(NO_3)_3.3H_2O$, rhodium triformate, rhodium triacetate, rhodium trinaphthenate, dirhodium octacarbonyl, tetrarhodium dodecacarbonyl, hexarhodium hexadecacarbonyl, rhodium dicarbonyl acetyl acetonate, $Rh(C_5H_5N)_3Cl_3$, $RhBr[(C_6H_5)_3P]_3$, $RhCl[(C_6H_5)_3P]_3H_2$, $Rh(CO)Cl[(C_6H_5)_3P]_2$, $Rh(CO_4)I_2$ and $Rh(CO)[(C_6H_5)_3As]_2$. A very suitable rhodium compound is $RhCl_3.3H_2O$. The rhodium compound may optionally be used as a dispersion on a carrier, for instance coke, aluminium oxide or silica gel.

The quantity of rhodium compound advantageously lies between $3.10^{-6}$ and $10^{-2}$, in particular between $10^{-5}$ and $10^{-2}$ and most preferably between $3.10^{-5}$ and $3.10^{-3}$, gram atom rhodium per mol alcohol. For instance, very good results are obtained using about $4.10^{-4}$ gram atom rhodium per mol alcohol.

The iodide and/or bromide source may be for instance elemental iodine or bromine, or hydrogen iodide or hydrogen bromide or a compound $R^4I$, $R^4Br$, $R^4COI$ or $R^4COBr$, wherein $R^4$ represents an alkyl group or an aralkyl group which is optionally substituted with bromium or iodine and preferably has not more than 12 carbon atoms. Compounds $R^4I$ or $R^4COI$, wherein $R^4$ represents an alkyl group with 1—4 carbon atoms, in particular methyl iodide, are especially preferred as iodide sources. Specific examples of other suitable iodide and/or bromide sources are $CH_3Br$, $C_2H_5I$, $C_4H_9I$, $C_8H_{17}I$, $CH_2I_2$, $C_2H_4I_2$, $CH_2IBr$, $CHI_3$ and $C_2H_4IBr$. Ammonium, phosphonium, arsonium and stibonium iodides and bromides may also be used as the iodide and/or bromide source.

Examples of such compounds are triphenylphosphonium iodide, methyltriphenylphosphonium iodide, tetramethylammonium iodide, tetraisopropylammonium iodide, tetrabutylammonium bromide and tetrabutylammonium iodide. If the catalyst contains an iodine or bromine compound of rhodium, this may also act as the iodide or bromide source.

The quantity of iodide and/or bromide source, viz. the total number of gram atoms I and/or Br present in the reaction mixture, generally lies between 1 and 1000, preferably between 1 and 500 — and in particular between 10 and 300, gram atoms I and/or Br per gram atom rhodium.

The hydrocarbon groups $R^1$, $R^2$ and $R^3$ which may be present in the compound of formula I to be used as promoter in the process according to the invention may be alkyl, cycloalkyl, aryl, aralkyl or alkaryl groups which advantageously have not more than 30, in particular not more than 20, carbon atoms and optionally have been substituted with one or more substituents, such as for instance halogen atoms or a group

$$R^5R^6X=Y,$$

wherein X and Y have the meanings mentioned hereinbefore and $R^5$ and $R^6$ each represent an unsubstituted or substituted hydrocarbon group. If $R^2$ and $R^3$ together with X form a heterocyclic group, the latter advantageously contains not more than 20 carbon atoms. Specific examples are the phospholane, the phosphorinane and the phosphohepane groups wherein the groups $R^2$ and $R^3$ together represent an alkylene group having 4, 5 or 6 carbon atoms, respectively, and the 9-phosphabicyclo [4.2.1] nonane group and the 9-phosphabicyclo [3.3.1] nonane group. These heterocyclic groups may be substituted for instance with hydrocarbon groups.

Compounds of formula I wherein a and b are O, X is phosphorus and Y is oxygen or sulphur and $R^1$, $R^2$ and $R^3$ represent alkyl groups having 1—12 carbon atoms or cylcoalkyl, aryl, aralkyl or alkaryl groups having 5—12 carbon atoms are preferred. Especially preferred are compounds of general formula I wherein Y represents oxygen and $R^1$, $R^2$ and $R^3$ represent alkyl groups having 1—12 carbon atoms, or phenyl groups.

Specific examples of compounds of formula I are the oxides, sulphides or selenides of secondary and tertiary phophines, arsines and stibines, such as trimethylphosphine oxide, diethylphosphine oxide, triethylphosphine oxide, tri-n-butylphosphine oxide, trioctylphosphine oxide, diphenylphosphine oxide,

tri-p-tolylphosphine oxide, tricyclo-hexylphosphine oxide, diphenylethylphosphine oxide, tri(1-naphthyl)-phosphine oxide, trimethylphosphine sulphide, tri-4-chlorophenylphosphine sulphide, triphenylphosphine sulphide, tricyclohexylphosphine sulphide, tri-n-butylphosphine sulphide, triphenylphosphine selenide, tris(1-naphthyl)-phosphine selenide, triethylarsine oxide, triphenylstibine oxide and triphenylarsine sulphide. Triphenylphosphine sulphide and particularly triphenylphosphine oxide are very useful promoters. Specific examples of compounds with a heterocyclic phosphorus-containing group are 1-phenylphospholane oxide, 1-phenylphosphorinane oxide, 9-phenyl-9-phosphabicyclo [4.2.1] nonane oxide, 9-phenyl-9-phosphabicyclo [3.3.1]-nonane oxide, 9-eicosyl-9-phosphabicyclo [4.2.1] nonane oxide, 9-eicosyl-9-phosphabicyclo [3.3.1] nonane oxide, 1-phenylphospholane sulphide, 1-phenylphosphorinane sulphide.

Examples of compounds of general formula I wherein a and/or b are 1 are the alkyl, cycloalkyl, aryl, aralkyl or alkaryl esters of phosphonic acids and phosphinic acids and the analogues of these compounds in which the double bonded oxygen atom has been replaced by a sulphur or selenium atom and/or the phosphorus atom by an arsenic or antimony atom. Specific examples of such compounds are dimethylmethyl phosphonate, diethylmethyl phosphonate, diphenylmethyl phosphonate, methyldiethyl phosphinate and phenyldimethyl phosphinate.

Specific examples of compounds of formula I wherein one or more of the groups $R^1$, $R^2$ and $R^3$ are substituted with a group

$$R^5R^6X=Y$$

are the compounds:

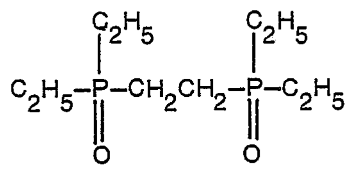 and 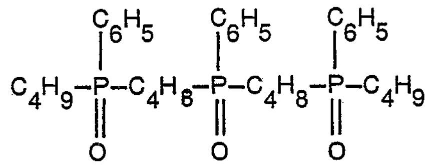

Finally, in the process according to the invention use may be made of complexes obtained by reaction of a compound of formula I with a hydrocarbon iodide or bromide, such as for instance a $CH_3I$, an acyl iodide or bromide, or hydrogen iodide or bromide.

Examples of such complexes are: $[(C_6H_5)_3PO—H—OP(C_6H_5)_3]^+I_3^-$ and $[(C_2H_5)_3AsO—H—OAs(C_2H_5)_3]^+I^-$. It has been found that such complexes, which may probably also be formed in situ by reaction of the compound of formula I with iodine or bromine compounds present in the reaction mixture, are very active promoters in the process according to the invention.

If in the compound of formula I X represents phosphorus and Y oxygen and a and b are 0, this compound may be formed in situ by using not the relevant compound of formula I, but the corresponding phosphine and carrying out the reaction in the presence of molecular oxygen or hydrogen peroxide.

The quantity of compound of formula I used as promoter in the process according to the invention may vary within wide limits, for instance between 0.1 and 300 mol per gram atom rhodium. Preference is given to the use of 1—200, in particular 10—100, mol per gram atom rhodium.

The process according to the invention is preferably carried out a temperature between 110 and 225°C, in particular between 125 and 200°C. The reaction is generally carried out at a partial CO pressure between 0.5 and 70 bar. High pressures, up to for instance 1000 bar may be used if desired, but in general they are unattractive for technical and economic reasons.

The carbon monoxide used in the process according to the invention may optionally be mixed with other gases, such as for instance carbon dioxide, methane, nitrogen or noble gases. Generally the presence of hydrogen is less desirable. Preference is given to the use of carbon monoxide or a carbon monoxide-containing gas containing less than 5%v hydrogen.

The process may be carried out in the liquid phase or in the gaseous phase, preference being given to the liquid phase. Usually there is no need for the use of an (additional) solvent since the alcohol used as starting material has a sufficient degree of solvent activity. Other components of the reaction mixture, for instance a liquid iodide source, such as for instance $CH_3I$, or the ester formed or the carboxylic acid formed, may also be active as solvents. If desired, additional quantities of these compounds may be added to the reaction mixture. Suitable (additional) solvents are for instance acetic acid, propionic acid, methyl acetate, butyrolactone, dimethyl ether, diethyl ether, acetic anhydride, methyl t.butyl ether, diglyme, tetraglyme, tetrahydrofuran, 1,4-dioxane, 1,3-dioxane, dimethyl sulphone, diethyl sulphone, methylethyl sulphone, methylbutyl sulphone, sulpholane, 2-methyl sulpholane, 3-methyl sulpholane, 2-methyl-4-butyl sulpholane, dimethyl sulphoxide, diethyl sulphoxide and N-methyl pyrolidone. The solvent may optionally have promoter activity, viz. it may have a favourable influence on the activity and/or selectivity of the catalyst system. If desired, other promoters such as for instance phosphines or metal compounds, for instance compounds of other Group VIII metal, may be added to the reaction mixture as well. Water may also be present; this will generally raise the degree of formation of the carboxylic acid in relation to the ester.

The process according to the invention may be carried out continuously or batch-wise. The reaction mixture obtained may be worked-up with the aid of known techniques, such as fractional distillation.

Furthermore, the process may be integrated into existing processes for preparing the starting materials or for further processing the carboxylic acid obtained or the ester obtained.

Example 1

A magnetically stirred 300 ml Hastelloy C autoclave (Hastelloy is a trade mark) was charged with 1.25 mol methanol (50 ml) and 0.167 mol acetic acid and the quantities given in Table A of $RhCl_3.3H_2O$, methyl iodide and triphenylphosphine oxide. The autoclave was flushed with carbon monoxide and filled with carbon monoxide at a pressure of 30 bar and then heated to 170°C. After a reaction time of 3.5 hours the reaction mixture was cooled and analysed by gas-liquid chromatography. The quantities of acetic acid and methyl acetate were determined and the total quantity of acetic acid formed by carbonylation of methanol was computed. Table A gives the carbonylation rates expressed in grams of acetic acid formed per gram of rhodium per gram of iodine per hour. This method of expression is the most correct one when determining the activity of the catalyst system, since generally the reaction rate is directly proportional to both the quantity of rhodium and the quantity of I present in the reaction mixture.

TABLE A

| $RhCl_3.3H_2O$ rate (mmol) | Methyl iodide (mmol) | Triphenyl-phosphine oxide (mmol) | Number of moles carbonylated methanol | Carbonylation g acetic acid/ g Rh/g I/h |
|---|---|---|---|---|
| 0.25 | 17 | — | 0.20 | 66 |
| 0.25 | 17 | 4 | 0.28 | 90 |
| 0.25 | 17 | 8 | 0.31 | 100 |
| 0.1 | 34 | 8 | 0.30 | 123 |

Table A clearly shows the strong promoter action of triphenylphosphine oxide.

Example II

With the object of studying the carbonylation reaction under conditions prevailing at high methanol conversion (the presence of a large quantity of acetic acid) the experiment of Example I was repeated with the autoclave containing 0.25 mol methanol, 0.83 mol acetic acid, 0.1 mmol $RhCl_3.3H_2O$ and the quantities given in Table B of methyl iodide, triphenylphosphine oxide and triphenylphosphine.

TABLE B

| Methyl iodide (mmol) | Triphenyl-phosphine oxide (mmol) | Triphenyl phosphine (mmol) | Number of moles carbonylated methanol | Carbonylation rate g acetic acid/ g Rh/g I/h |
|---|---|---|---|---|
| 34 | — | — | 0.07 | 27 |
| 34 | 8 | — | 0.16 | 65 |
| 17 | 8 | — | 0.075 | 70* |
| 34 | — | 8 | 0.12 | 56* |

* In these experiments the reaction time was 3h and the carbon monoxide pressure 40 bar.

Table B shows that triphenylphosphine oxide has a strong promoter action even in the presence of large quantities of acetic acid, that this action does not decrease when the quantity of methyl iodide is halved, and that the promoter action of triphenylphosphine is inferior to that of triphenylphosphine oxide.

Further, the analysis of the reaction mixture by gas-liquid chromatography has shown that the triphenylphosphine oxide had not decomposed during the reaction, whereas about 50% of the triphenylphosphine has been converted into a variety of unidentified compounds.

Example III

The experiment of Example I was repeated with the autoclave containing 0.25 mol methanol, 0.83 mol acetic acid, 0.2 mmol $RhCl_3.3H_2O$, 16 mmol triphenylphosphine oxide and 17 mmol methyl iodide. Before the autoclave was heated the carbon monoxide pressure was 40 bar. After a reaction time of 1.5 h it was found that 0.14 mol methanol had been carbonylated. The carbonylation rate was 130 g acetic acid/g Rh/g l/h.

Repetition of the experiment using 16 mmol triphenylphosphine instead of 16 mmol triphenylphosphine oxide, showed that after 1.5 h only 0.05 mol methanol had been carbonylated. The carbonylation rate was 46.5 g acetic acid/g Rh/g l/h.

This experiment shows that at low iodine concentrations triphenyl-phosphine oxide is a much better promoter than triphenylphosphine.

Example IV

a) Preparation of $[Rh(OCOCH_3)_2]_2.2CH_3OH$ according to UK patent specification No. 1,326,014.

5 g $RhCl_3.3H_2O$ and 10 g $CH_3COONa.3H_2O$ were dissolved in 100 ml acetic acid and 100 ml absolute ethanol. The solution was boiled for 1 hour under nitrogen using a reflux condenser. After cooling to room temperature a greenish precipitate formed; this was filtered off.

The raw product was dissolved in 600 ml boiling methanol, the solution was evaporated to 400 ml and cooled down to 0°C. The blue-green $[Rh(OCOCH_3)_2]_2.2CH_3OH$ crystals were filtered off and dried in vacuo.

b) Determination of catalytic activity of the complex prepared according to a).

The experiment of Example I was repeated with the autoclave containing 0.15 mmol of the complex prepared according to a), 0.25 mol methanol, 0.83 mol acetic acid, 8 mmol methyl iodide and 16 mmol triphenylphosphine oxide. The autoclave was flushed with carbon monoxide, filled with carbon monoxide at a pressure of 40 bar and then heated to 170°C. After a reaction time of 2 h 0.10 mol methanol had been carbonylated. The carbonylation rate was 98 g acetic acid/g Rh/g l/h.

c) Carbonylation of methanol in the manner disclosed in UK patent specification No. 1,326,014.

A magnetically stirred 300 ml Hastelloy C autoclave was charged with 1.25 mol methanol (50 ml), 5 mmol $CH_3I$, 0.074 mmol $[Rh(OCOCH_3)_2]_2.2CH_3OH$, 0.3 mmol triphenylphosphine and 0.3 mmol $HBF_4$ (as a 60% aqueous solution). The autoclave was flushed with carbon monoxide, filled with carbon monoxide at a pressure of 50 bar and then heated to 100°C. After a reaction time of 3 h the reaction mixture was analysed by gas-liquid chromatography. There was found to be present only 1.6 mmol methyl acetate.

## Claims

1. A process for the preparation of carboxylic acids and/or esters by reaction of an alcohol with carbon monoxide in the presence of a rhodium compound, an iodide and/or bromide source and a phosphorus, arsenic or antimony-containing compound as promoter, characterized in that the reaction is carried out in the presence of a compound of the formula

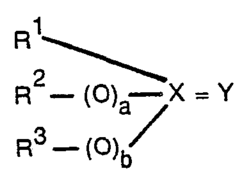

I

wherein X represents phosphorus, arsenic or antimony and Y oxygen, sulphur or selenium, and either a and b, independent of one another, are 0 or 1, $R^1$ represents hydrogen or an unsubstituted or substituted hydrocarbon group and $R^2$ and $R^3$ each represent an unsubstituted or substituted hydrocarbon group, or a and b are 0 and $R^2$ and $R^3$ together with X form a heterocyclic group and $R^1$ represents hydrogen or an unsubstituted or substituted hydrocarbon group, or in the presence of a complex of a compound of formula I with a hydrocarbon iodide or bromide, an acyl iodide or bromide or hydrogen iodide or bromide.

2. A process as claimed in claim 1, characterized in that the alcohol is a hydrocarbon substituted with one or more hydroxyl groups and having 1—20 carbon atoms, in particular an alkanol having 1—12 carbon atoms.

3. A process as claimed in claim 1 or 2, characterized in that the iodide and/or bromide source is elemental iodine or bromine, hydrogen iodide, hydrogen bromide or a compound $R^4I$, $R^4Br$, $R^4COI$ or $R^4COBr$, wherein $R^4$ represents an alkyl group optionally substituted with bromine or iodine or an aralkyl group preferably having not more than 12 carbon atoms.

4. A process as claimed in claims 1—3, characterized in that the total number of gram atoms of I and/or Br present in the reaction mixture lies between 1 and 500 gram atoms I and/or Br per gram atom rhodium.

5. A process as claimed in claim 1—4, characterized in that the hydrocarbon groups $R^1$, $R^2$ and $R^3$ are alkyl, cycloalkyl, aryl, aralkyl or alkaryl groups having not more than 30 carbon atoms.

6. A process as claimed in claims 1—4, characterized in that $R^1$ and $R^2$ together with X form a heterocyclic group having not more than 20 carbon atoms.

7. A process as claimed in claims 1—5, characterized in that a and b are O, X is phosphorus and Y is oxygen or sulphur and $R^1$, $R^2$ and $R^3$ represent alkyl groups having 1—12 carbon atoms or cycloalkyl, aryl, aralkyl or alkaryl groups having 5—12 carbon atoms.

8. A process as claimed in claim 7, characterized in that Y is oxygen and $R^1$, $R^2$ and $R^3$ represent alkyl groups having 1—12 carbon atoms, or phenyl groups.

9. A process as claimed in claims 1—8, characterized in that the reaction is carried out at a temperature lying between 110 and 225°C, in particular between 125 and 200°C.

**Patentansprüche**

1. Verfahren zur Herstellung von Carbonsäuren und/oder-estern durch Umsetzung eines Alkohols mit Kohlenmonoxid in Gegenwart einer Rhodiumverbindung, einer Iodid- und/oder Bromidquelle und einer Phosphor-/Arsen oder Antimon enthaltenden Verbindung als Promotor, dadurch gekennzeichnet, daß die Reaktion durchgeführt wird in Gegenwart einer Verbindung der Formel

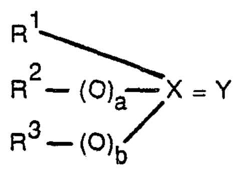

I

in der X Phosphor, Arsen oder Antimon, Y Sauerstoff, Schwefel oder Selen bedeutet, und entweder a und b unabhängig voneinander 0 oder 1 sind, $R^1$ Wasserstoff oder eine nicht substituierte oder substituierte Kohlenwasserstoffgruppe bedeutet, und $R^2$ und $R^3$ jeweils eine unsubstituierte oder substituierte Kohlenwasserstoffgruppe bedeuten, oder a und b 0 sind und $R^2$ und $R^3$ zusammen mit X eine heterocyclische Gruppe bilden und $R^1$ Wasserstoff oder eine unsubstituierte oder substituierte Kohlenwasserstoffgruppe bedeutet, oder in Gegenwart eines Komplexes einer Verbindung der Formel 1 mit einem Kohlenwasserstoffiodid oder -bromid, einem Acyliodid oder bromid oder Iod- oder Bromwasserstoff.

2. Verfahren nach Anspruch 1, dadurch gekennzeichnet, daß der Alkohol ein durch eine oder mehrere Hydroxylgruppen substituierter Kohlenwasserstoff mit 1 bis 20 Kohlenstoffatomen, besonders ein Alkanol mit 1 bis 12 Kohlenstoffatomen ist.

3. Verfahren nach Anspruch 1 oder 2, dadurch gekennzeichnet, daß die Iodid- und/oder Bromidquelle elementares Iod oder Brom, Iodwasserstoff, Bromwasserstoff oder eine Verbindung $R^4I$, $R^4Br$, $R^4COI$ oder $R^4COBr$ ist, wobei $R^4$ eine gegebenenfalls durch Brom oder Iod substituierte Alkyl- oder eine Aralkylgruppe mit vorzugsweise nicht mehr als 12 Kohlenstoffatomen ist.

4. Verfahren nach den Ansprüchen 1 bis 3, dadurch gekennzeichnet, daß die Gesamtzahl g-Atome I und/oder Br in dem Reaktionsgemisch zwischen 1 und 500 g-Atome I und/oder Br pro g-Atom Rhodium liegt.

5. Verfahren nach den Ansprüchen 1 bis 4, dadurch gekennzeichnet, daß die Kohlenwasserstoffgruppen $R^1$, $R^2$ und $R^3$ Alkyl-, Cycloalkyl-, Aryl-, Aralkyl- oder Alkarylgruppen mit nicht mehr als 30 Kohlenstoffatomen sind.

6. Verfahren nach den Ansprüchen 1 bis 4, dadurch gekennzeichnet, daß $R^1$ und $R^2$ zusammen mit X eine heterocyclische Gruppe mit nicht mehr als 20 Kohlenstoffatomen bilden.

7. Verfahren nach den Ansprüchen 1 bis 5, dadurch gekennzeichnet, daß a und b 0 sind, X Phosphor und Y Sauerstoff oder Schwefel ist, $R^1$, $R^2$ und $R^3$ Alkylgruppen mit 1 bis 12 Kohlenstoffatomen oder Cycloalkyl-, Aryl-, Aralkyl- oder Alkarylgruppen mit 5 bis 12 Kohlenstoffatomen bedeuten.

8. Verfahren nach Anspruch 7, dadurch gekennzeichnet, daß Y Sauerstoff ist, und $R^1$, $R^2$ und $R^3$ Alkylgruppen mit 1 bis 12 Kohlenstoffatomen oder Phenylgruppen bedeuten.

9. Verfahren nach Anspruch 1 bis 8, dadurch gekennzeichnet, daß die Reaktion bei einer Temperatur zwischen 110 und 225°C, insbesondere zwischen 125 und 200°C durchgeführt wird.

**Revendications**

1. Un procédé pour la préparation d'acides carboxyliques et/ou d'esters par réaction d'un alcool avec l'oxyde de carbone en présence d'un composé du rhodium, d'une source d'iodure et/ou de bromure et d'un composé contenant du phosphore, de l'arsenic ou de l'antimoine comme promoteur, caractérisé en ce que la réaction est conduite en présence d'un composé de la formule

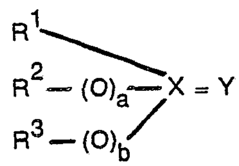

I

où X représente du phosphore, de l'arsenic ou de l'antimoine et Y de l'oxygène, du soufre ou du sélénium et soit a et b, indépendamment l'un de l'autre, sont 0 ou 1, $R^1$ représente de l'hydrogène ou un groupe d'hydrocarbure substitué ou non et $R^2$ et $R^3$ représentent chacun un groupe d'hydrocarbure substitué ou non, soit a et b sont 0 et $R^2$ et $R^3$ en même temps que X forment un groupe hétérocyclique et $R^1$ représente de l'hydrogène ou un groupe d'hydrocarbure substitué ou non, ou en présence d'un complexe d'un composé de formule I avec un iodure ou bromure d'hydrocarbure, un iodure ou bromure d'acyle ou le bromure ou l'iodure d'hydrogène.

2. Un procédé selon la revendication 1, caractérisé en ce que l'alcool est un hydrocarbure substitué par un ou plusieurs groupes hydroxyle et ayant 1—20 atomes de carbone, en particulier un alcanol ayant 1—12 atomes de carbone.

3. Un procédé selon la revendication 1 ou 2, caractérisé en ce que la source d'iodure et/ou de bromure est de l'iode ou du brome élémentaire, de l'iodure d'hydrogène, du bromure d'hydrogène ou un composé $R^4I$, $R^4Br$, $R^4COI$ ou $R^4COBr$, où $R^4$ représente un groupe alcoyle éventuellement substitué par du brome ou de l'iode ou un groupe aralcoyle n'ayant de préférence pas plus de 12 atomes de carbone.

4. Un procédé selon les revendications 1—3, caractérisé en ce que le nombre total d'atomes-grammes de I et/ou de Br présents dans le mélange réactionnel est compris entre 1 et 500 atomes-grammes de I et/ou de Br par atome-gramme de rhodium.

5. Un procédé selon les revendications 1—4, caractérisé en ce que les groupes d'hydrocarbures $R^1$, $R^2$ et $R^3$ sont des groupes alcoyle, cycloalcoyle, aryle, aralcoyle ou alcaryle n'ayant pas plus de 30 atomes de carbone.

6. Un procédé selon les revendications 1—4, caractérisé en ce que $R^1$ et $R^2$ en même temps que X forment un groupe hétérocyclique n'ayant pas plus de 20 atomes de carbone.

7. Un procédé selon les revendications 1—5, caractérisé en ce que a et b sont 0, X est du phosphore et Y est de l'oxygène ou du soufre et $R^1$, $R^2$ et $R^3$ représentent des groupes alcoyle ayant 1—12 atomes de carbone ou des groupes cycloalcoyle, aryle, aralcoyle ou alcaryle ayant 5—12 atomes de carbone.

8. Un procédé selon la revendication 7, caractérisé en ce que Y est de l'oxygène et $R^1$, $R^2$ et $R^3$ représentent des groupes alcoyle ayant 1—12 atomes de carbone ou des groupes phényle.

9. Un procédé selon les revendications 1—8, caractérisé en ce que la réaction est conduite à une température comprise entre 110 et 225°C, en particulier entre 125 et 200°C.